Europäisches Patentamt

European Patent Office

Office européen des brevets

(11) Veröffentlichungsnummer: **0 470 520 A2**

## EUROPÄISCHE PATENTANMELDUNG

(21) Anmeldenummer: **91113030.0**

(51) Int. Cl.⁵: **C12P 21/02**, //C12N15/40

(22) Anmeldetag: **02.08.91**

(30) Priorität: **07.08.90 DE 4025020**
**20.07.91 DE 4124132**

(43) Veröffentlichungstag der Anmeldung:
**12.02.92 Patentblatt 92/07**

(84) Benannte Vertragsstaaten:
**AT CH DE DK FR GB IT LI NL SE**

(71) Anmelder: **Bendzko, Peter, Dr.**
**Ifflandstrasse 32**
**O-1147 Berlin(DE)**

Anmelder: **Maximov, Ewgenij, Dr.**
**Hans-Loch-Strasse 64 e**
**O-1136 Berlin(DE)**

Anmelder: **Fischer, Karl-Heinz**
**Am Kochberg 14**
**W-6457 Maintal 3(DE)**

(72) Erfinder: **Bendzko, Peter, Dr.**
**Ifflandstrasse 32**
**O-1147 Berlin(DE)**
Erfinder: **Maximov, Ewgenij, Dr.**
**Hans-Loch-Strasse 64 e**
**O-1136 Berlin(DE)**
Erfinder: **Fischer, Karl-Heinz**
**Am Kochberg 14**
**W-6457 Maintal 3(DE)**

(74) Vertreter: **Jochem, Bernd, Dipl.-Wirtsch.-Ing.**
**Patentanwälte Beyer & Jochem Postfach 17**
**01 45 Staufenstrasse 36**
**W-6000 Frankfurt/Main 1(DE)**

(54) Verfahren zur Herstellung von Peptiden und Polypeptiden in einem zellfreien Translationssystem.

(57) Das Verfahren dient zur Herstellung von Oligo- und Polypeptiden in einem zellfreien Translationssystem, welches neben einer Pufferlösung Prozeßkomponenten enthält, die an den ribosomalen Translationsprozessen in der Initiations-, Elongations- und/oder Terminationsphase beteiligt sind. Um die Syntheseleistung zu verbessern, ist vorgesehen, daß die einzelnen ribosomalen Translationsprozesse phasengerichtet gesteuert und wenigstens teilweise synchronisiert werden durch in zeitlichen Abständen erfolgende Konzentrationsänderungen von wenigstens einer der Prozeßkomponenten. Dies geschieht in der Weise, daß während wenigstens zwei der drei Phasen hinsichtlich wenigstens einer Prozeßkomponente für den Ablauf der jeweiligen Phase günstigere Bedingungen vorherrschen als in wenigstens einer der beiden anderen Phasen.

EP 0 470 520 A2

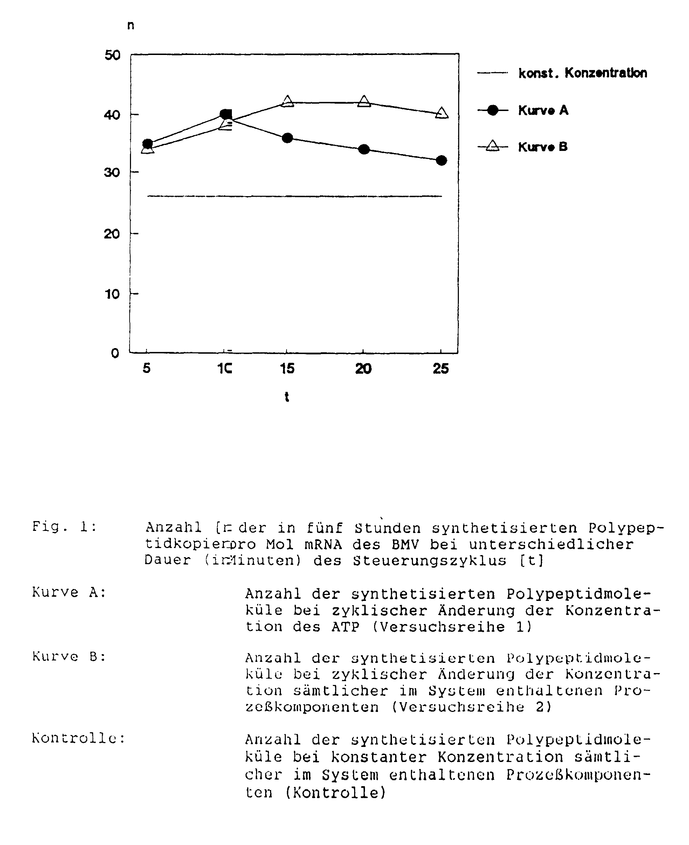

Fig. 1:     Anzahl [n] der in fünf Stunden synthetisierten Polypep-
            tidkopien pro Mol mRNA des BMV bei unterschiedlicher
            Dauer (in Minuten) des Steuerungszyklus [t]

Kurve A:                    Anzahl der synthetisierten Polypeptidmole-
                            küle bei zyklischer Änderung der Konzentra-
                            tion des ATP (Versuchsreihe 1)

Kurve B:                    Anzahl der synthetisierten Polypeptidmole-
                            küle bei zyklischer Änderung der Konzentra-
                            tion sämtlicher im System enthaltenen Pro-
                            zeßkomponenten (Versuchsreihe 2)

Kontrolle:                  Anzahl der synthetisierten Polypeptidmole-
                            küle bei konstanter Konzentration sämtli-
                            cher im System enthaltenen Prozeßkomponen-
                            ten (Kontrolle)

Die vorliegende Erfindung bezieht sich auf Verfahren zur Herstellung von Peptiden, nämlich Oligo- und Polypeptiden in einem zellfreien Translationssystem, welches neben einer Pufferlösung Prozeßkomponenten enthält, die an den einzelnen, jeweils eine Initiations-, Elongations- und Terminationsphase umfassenden ribosomalen Translationsprozessen im System unmittelbar oder mittelbar beteiligt sind.

Es ist bekannt, daß die genannten Oligo- und Polypeptide in der Medizin als Bioregulatoren der biologischen Prozesse, z.B. als Peptidaktivatoren des Immunsystems, Polypeptid-Neuromediatoren und Transmitter, Polypeptid-Regulatoren des Elektrolytstoffwechsels etc. verwendet werden können. Es ist ferner bekannt, daß Oligo- und Polypeptide auch in der Landwirtschaft als Biostimulatoren, z.B. Wachstumshormone bzw. Leistungsförderer, in der Bioelektronik und Informationstechnologie verwendet werden können.

Zur Herstellung von Oligo- und Polypeptiden sind zwei Verfahren bekannt, die chemische Synthese und das Verfahren der Gentechnik.

Das Verfahren der chemischen Synthese wird zur großtechnischen Produktion von Peptiden mit relativ geringer Länge (15 bis 20 Aminosäurereste) und nur in Ausnahmefällen zur Synthese von Peptiden größerer Länge angewendet. Je größer nämlich die zu synthetisierende Peptidkette ist, desto geringer ist die Peptidausbeute. In dem Maße, wie die Peptidausbeute mit exponentieller Tendenz abnimmt, nehmen Verunreinigungen zu, welche nur schwer und unter hohem Kostenaufwand zu entfernen sind. Die Probleme sind darauf zurückzuführen, daß die Aminosäurereste am $\alpha$-C-Atom racemisieren, die Polypeptidketten häufig nur unvollständig verlängert werden und überdies Schwierigkeiten bei der Wahl geeigneter Schutzgruppen wie auch bei deren Abspaltung entstehen.

Das Verfahren der Gentechnik für die präparative Speicherung von Polypeptiden ist ebenfalls in der Anwendung begrenzt. Dies ist auf die mit der Aussonderung des Expressionsproduktes mit Hilfe der transformierten Zellen verbundenen Schwierigkeiten, auf die durch einige Endprodukte verursachte Letalität der Produzentenzelle, auf die Ausscheidung der transformierten Plasmide aus einer Zelle und auf die proteolytische Degradation des Expressionproduktes eines fremden Gens zurückzuführen. Letzteres verursacht besondere Schwierigkeiten bei der Aussonderung vieler Polypeptide, insbesondere der Polypeptide mit einer Länge bis zu 60 Aminosäureresten, die sich infolge des Fehlens kompakter Struktur durch die intra- und extrazellulären Proteasen abbauen lassen.

Hieraus geht hervor, daß die bekannten Verfahren zur Produktion von Oligo- und Polypeptiden für die Erzeugung von Peptiden mit einer Länge von 15 bis 60 Aminosäureresten praktisch nicht anwendbar sind. Andererseits ist aber bekannt, daß gerade viele biologisch aktive Polypeptide, wie z.B. Peptidaktivatoren des Immunsystems, Peptid-Neuromediatoren und -Transmitter sowie Peptidaktivatoren des Elektrolytstoffwechsels eine solche Länge aufweisen.

Ein weiteres Verfahren zur Synthese von Peptiden basiert auf der Anwendung zellfreier Translationssysteme. Es sind eine ganze Reihe von zellfreien Translationssystemen auf der Grundlage von Zellextrakten aus verschiedenen Prokaryonten und Eukaryonten entwickelt worden. So ist beispielsweise ein Verfahren zur Erzeugung von Polypeptiden bekannt, wonach in einem zellfreien Translationssystem aus Weizenkeimen die Synthese des Globin-Polypeptids eines Kaninchens durchgeführt worden ist (Proc.Nat.Acad.Sci.USA Vol.70, 1973).

Das zellfreie Translationsystem stellt einen Zellextrakt dar, der keine groben zellulären Bestandteile mehr enthält. Zellfreie Translationssysteme werden in präparativen Mengen von der Firma SERVA angeboten.

Die Komponenten zellfreier Translationssysteme sind ausführlich in der Literatur beschrieben (vgl. The Eucaryotic Ribosome, e.d. by H.Bielka, Akademie-Verlag Berlin, 1982). Zu den unmittelbar am Translationsprozeß beteiligten Komponenten zählen Ribosomen, ribosomale Untereinheiten, Proteinfaktoren (Initiations-, Elongations-, Terminations-Faktoren), Polynucleotide (mRNA, Aminoacyl-, Peptidyl-tRNA), Nucleosidtriphosphate (ATP, GTP), anorganische Ionen sowie auch Produkte, die während der Translation gebildet werden. Es ist bekannt, daß der in vivo-Translationsprozeß von einer Reihe weiterer Prozesse begleitet wird, z.B. der Transcription des genetischen Codes der entsprechenden mRNA des zu synthetisierenden Produktes, der Synthese der Aminoacyl-tRNA, der Nucleotidtriphosphat-Regenerierung, der enzymatischen Spaltung von Polypeptiden und Polynucleotiden, dem Prozeß der Inhibierung der Translation oder einzelner Enzyme bzw. der Aktivierung des Prozesses durch eine Reihe von Aktivatoren (wie Spermidin, Hemin, organische Lösungsmittel u.a.).

Aus der EP A1 0312617 ist ein Verfahren zur Herstellung von Polypeptiden bekannt, wobei die Polypeptide in einem zellfreien Translationssystem an Ribosomen erhalten werden, das als Substrate ATP, GTP und Aminosäuren enthält, und wobei die Produkte der Translation im System entstehen, das die Endprodukte AMP, GDP, Pyrophosphat und anorganisches Phosphat enthält. Bei diesem Verfahren werden während der Translation aus dem System in dem Maße, in dem die Substrate unter Bildung der gewünschten Produkte verbraucht werden, die Produkte der Translation, die AMP, GDP, Pyrophosphat, anorganisches Phosphat und das Endprodukt enthalten, herausgeführt und gleichzeitig in das System Substrate in Form von Aminosäuren, ATP und GTP zur Aufrechterhaltung ihrer Ausgangskonzentrationen eingeführt.

Wesentliches Merkmal dieses Verfahrens ist, daß die Konzentration der Ausgangssubstrate während des gesamten Verfahrens konstant gehalten wird, und daß mittels Einsatzes einer Ultrafiltrationszelle auch die Konzentration aller übrigen Komponenten konstant gehalten wird.

In keinem der bekannten Verfahren findet jedoch die Tatsache Berücksichtigung, daß der an einem einzelnen Ribosom stattfindende Translationsprozeß aus drei verschiedenen Phasen, nämlich der Initiations-, Elongations- und Terminationsphase, zusammengesetzt ist, wobei bekannt ist, daß die in vivo-Bedingungen, unter denen ein optimaler Ablauf der drei Phasen gewährleistet ist, jeweils voneinander abweichen.

Der vorliegenden Erfindung liegt daher die Aufgabe zugrunde, ein Verfahren der eingangs genannten Art zu schaffen, bei dem den wissenschaftlichen Erkenntnissen über den phasenweise ablaufenden Translationsprozeß Rechnung getragen wird und mit dessen Hilfe die Syntheseleistung gegenüber den bekannten Verfahren spürbar verbessert werden kann.

Vorstehende Aufgabe wird erfindungsgemäß dadurch gelöst, daß die einzelnen ribosomalen Translationsprozesse phasengerichtet gesteuert und wenigstens teilweise synchronisiert werden durch in zeitlichen Abständen erfolgende Konzentrationsänderungen von wenigstens einer der Prozeßkomponenten, derart daß während wenigstens zwei der drei Phasen hinsichtlich wenigstens einer Prozeßkomponente für den Ablauf der jeweiligen Phase günstigere Bedingungen vorherrschen als in wenigstens einer der beiden anderen Phasen.

Es wurde herausgefunden, daß man die Ausbeute im zellfreien Translationssystem erheblich steigern kann, wenn man dem System die am Translationsprozeß beteiligten Komponenten je nach Phasenverlauf variierend zuführt.

Bei Kenntnis der ungefähren Synthesezeit für ein herzustellendes Peptid kann die in vitro-Translation durch im wesentlichen zu Beginn und am Ende des ribosomalen Translationsprozesses stattfindende Konzentrationsänderungen im System exakt gesteuert und auf diese Weise optimiert werden. Uneingeschränkt gilt dieses Prinzip allerdings nur für die Produktion von Peptiden in monoribosomalen Systemen. Wenn eine mRNA-Kette mehr als 60 bis 75 Nucleinsäuren enthält, so ist die Bildung eines polisomalen Systems möglich und damit kann die mRNA erneut initiieren, während die Kettenverlängerung noch im vollen Gange ist. Da Initiations- und Terminationsphase im Verhältnis zur Elongationsphase relativ kurz sind, können kurzzeitig infolge der Unterstützung von Initiation oder Termination auftretende suboptimale Bedingungen für im Stadium der Elongation befindliche Peptidketten in Kauf genommen werden. Der Zeitabstand zwischen dem Beginn zweier Initiationsoder Terminationsphasen ist in derartigen Fällen kürzer als die entsprechende Synthesezeit für die vollständige Translation eines Peptids. Da die Anzahl Ribosomen am konkreten Polysom elektronenmikroskopisch erkennbar und eine für eine bestimmte mRNA charakteristische Höchstzahl nicht überschritten wird, können bei Kenntnis der Syntheszeit für ein bestimmtes Peptid die Zeitpunkte für den Beginn der Steuerung der einzelnen Phasen durch einfache Division der ribosomalen Syntheszeit durch die Anzahl Ribosomen am Polysom ermittelt werden.

Für die jeweils maßgebliche Synthesezeit kommen je nach herzustellendem Oligo- oder Polypeptid Zeiten in einer Größenordnung von 0,07 Sekunden bis zu 7,5 Stunden in Betracht. Die kürzeste Synthesezeit von 0,07 Sekunden wurde als Mittelwert für die Synthese eines Dipeptids in einem prokaryotischen Translationssystem gemessen. Im Falle der Synthese von längeren Polypeptiden kann sich die Synthesegeschwindigkeit jedoch verringern. So beträgt beispielsweise die mittlere Synthesezeit für Calcitonin im zellfreien Translationssystem 15 Sekunden. Die längste Synthesezeit von 7,5 Stunden ergibt sich bei der Synthese der ribosomalen Proteine S10, L3, L4, L23, L2, L22, L19, S3, L16, L29, S19 einschließlich der intercistronen Bereiche, welche durch eine der längsten polycistronischen Ribonucleinsäuren codiert werden. Dazwischen liegen Zeiten, die entweder mit der Translation an kürzeren Matrizen oder der Ausbildung von polyribosomalen Translationskomplexen verknüpft sind.

Experimentell kann die Synthesezeit eines bestimmten Translationsprozesses folgenderweise festgestellt werden: Man nimmt ein vollständiges Translationssystem, aber ohne Aminosäuren. Zum Startzeitpunkt fügt man alle für die Proteinsynthese notwendigen Aminosäuren hinzu, und zwar unter Verwendung einer radioaktiv markierten Aminosäure. In diesem Moment bildet sich der Initiationskomplex und danach starten die Ribosome die Proteinsynthese. Dabei ist zu beachten, daß die Synthesezeit für Aminoacyl-mRNA unvergleichbar kürzer ist als die des gesamten Translationsprozesses. In bestimmten Zeitabständen entnimmt man aus dem Reaktionsgemisch Aliquoten. Der Prozeß wird durch kurzzeitige Erhitzung oder Bearbeitung mit Sodiumdodecylsulfat unterbrochen. Um das Syntheseprodukt von nicht vollsynthetisierten Ketten zu trennen, wird eine Gelelektrophorese durchgeführt. Die Radioaktivität des Syntheseprodukts wird gemessen und in dessen molare Menge umgerechnet. Die Zeit, welche für die Synthese von 0,5 Mol Syntheseprodukt pro Mol mRNA notwendig ist, wird als durchschnittliche Synthesezeit in diesem Translationsprozeß gewertet. Unter Zugrundelegung der ermittelten Werte kann der Prozeß anschließend weiter optimiert werden, beispielsweise wie weiter unten beschrieben (Versuchsreihe 1 und 2).

Vor allem wenn Initiationsphasen durch geeignete Maßnahmen unterstützt werden, können die im zellfreien Translationssystem stattfindenden Translationsprozesse weitgehender synchronisiert werden. Mithin ist es auch möglich, den Steuerungszyklus so zu gestalten, daß jeweils die der Steuerung dienenden Maßnahmen nur zu jeder zweiten, dritten etc. Initiatins-, Elongations- bzw. Terminationsphase vorgenommen werden.

Jede der genannten Phasen des Translationsprozesses erfordert unterschiedliche Bedingungen für eine optimale Syntheseleistung. Für die Phase der Initiation, welche mit der Assoziation der ribosomalen Untereinheiten einhergeht, ist eine erhöhte Konzentration der Bestandteile des initiatorischen Komplexes, wie der Eiweißkomponenten des initiatorischen Komplexes, der entsprechenden mRNA, der ribosomalen Untereinheiten, der initiatorischen tRNA, ATP und einiger bivalenter anorganischer Kationen ($Mg^{++}$, $Ca^{++}$ u.a.) und gleichzeitig eine reduzierte Konzentration monovalenter anorganischer Kationen ($K^{+}$, $NH_4^{+}$ u.a.) vorteilhaft.

Die folgende Phase der Elongation erfordert gemäßigte Konzentrationen der bivalenten Kationen sowie die Einbeziehung von Elongationsfaktoren unter intensiver Zuführung von Aminoacyl-tRNA und GTP.

Die Phase der Termination, welche durch die Dissoziation der Ribosomen und die Freisetzung der synthetisierten Peptidkette charakterisiert ist, erfordert eine erhöhte Konzentration der Terminationsfaktoren, eine reduzierte Konzentration bivalenter Kationen sowie den Abtransport des Translationsproduktes.

Weiterhin können die enzymatischen Syntheseprozesse durch eine Reihe von Substanzen selektiv gesteuert werden, wozu insbesondere organische Moleküle, wie etwa organische Lösungsmittel (Ethanol, Glycerin, Dimethylformamid u.a.) in geringen Konzentrationen und Antibiotika zu zählen sind. Diese Substanzen können sowohl als Stimulatoren als auch als Inhibitoren in den unterschiedlichen Phasen des Translationsprozesses eingesetzt werden. Darüberhinaus sind eine Reihe weiterer molekularer Prozesse zu beachten, die nicht direkt mit der Translation der mRNA verknüpft sind, sie jedoch begleiten bzw. überlagern. Hierzu sind Inhibierung von Ribonucleasen und Proteasen sowie der Prozeß der Transcription zu zählen, welcher zur Synthese der mRNA führt. Dabei ist zu beachten, daß der Prozeß der Transcription wie schon der Prozeß der Translation zyklisch verläuft und ebenfalls die spezifisch von der Konzentration der beteiligten Komponenten beeinflußten Phasen der Initiation, Elongation und Termination umfaßt.

Es wird daher vorgeschlagen, zu Beginn des Prozesses der zellfreien Translation eine erhöhte Menge von Inhibitoren gegen überflüssige Ribonucleasen und Proteasen in das System zu geben und deren Konzentration in Abhängigkeit von der Stabilität des Inhibitor-Komplexes periodisch zu modifizieren.

Zusätzlich zur oder anstatt der selektiven Konzentrationsänderung einzelner am Translationsprozeß beteiligter Komponenten kann durch eine Erhöhung oder Reduzierung der Lösungsmittelkonzentration die Konzentration aller Komponenten in den einzelnen Phasen des Translationsprozesses geändert werden. Neben den anderen bereits aufgeführten Möglichkeiten kann auf diese Weise besonders die Assoziation der ribosomalen Untereinheiten in der Initiationsphase wie auch die Dissoziation des Terminationskomplexes in der Terminationsphase beeinflußt werden.

Zusammenfassend kann mit Hilfe des vorgeschlagenen Verfahrens eine Optimierung des gesamten Translationsprozesses auf dem Wege der Optimierung jeder einzelnen Prozeßstufe als Teil eines summarischen Prozesses erzielt werden. Die Optimierung der einzelnen Phasen der Translation wird dadurch erreicht, daß die Konzentration einer, vieler oder aller der am Prozeß der Translation oder der sie begleitenden Prozesse beteiligten Komponenten in zeitlichen Abständen geändert wird. Vorzugsweise finden die Konzentrationsänderungen im Rahmen eines sich regelmäßig wiederholenden Zyklus statt. Es überrascht in diesem Zusammenhang, daß bereits die zyklische Konzentrationsänderung von nur einer geeigneten Prozeßkomponente deutliche Auswirkungen auf die Peptidausbeute im Translationssystem haben kann.

Das vorgeschlagene Verfahren besitzt allerdings nicht nur den Vorzug, daß die Ausbeuten im zellfreien Translationssystem gegenüber den bekannten Verfahren erheblich höher ausfallen, es wird gleichzeitig erreicht, daß die einzelnen Translationsprozesse an jeder einzelnen mRNA weitgehender synchronisiert sind, so daß vor allem bei der Synthese langer Peptide der optimale Zeitpunkt für die Beendigung des Verfahrens gewählt werden kann und zwar unabhängig davon, ob das zellfreie Translationssystem diskontinuierlich oder kontinuierlich betrieben wird.

Zum Zwecke der Veranschaulichung der Erfindung wurden die folgenden Experimente, welche die Optimierung eines zellfreien Translationssystems auf der Basis des vorgeschlagenen Prinzips darstellen sollen, durchgeführt. Eine Beschränkung ist hieraus nicht herzuleiten.

Versuchsreihe 1: Variable Prozeßsteuerung durch zyklische Änderung der ATP-Konzentration

Es wurde der im nachfolgenden Absatz beschriebene Versuchsansatz durchgeführt:

Zu einem Milliliter eines zellfreien Translationssystems, welches 0,5 ml eines Weizenkeimextrakts, 2-10 µg eines Ribonucleaseinhibitors aus der Placenta des Menschen, und je 0,1 µg der Proteaseinhibitoren Pepstatin, Leupeptin und Aprotinin in einer Pufferlösung (25 mMol HEPES pH 7,6, 100 mMol Kaliumacetat, 2,5 mMol Magnesiumacetat, 40 mMol GTP, 5 mMol Dithiotreit, 1 % Glycerin, 20 mMol ($^3$H)Leucin und jeweils 20 mMol der übrigen 19 natürlichen Aminosäuren) enthielt, wurden 0,15 nMol mRNA-Virus BMV (Brome Mosaic Virus) gegeben und in einer 3 ml fassenden Standard-Ultrafiltrationszelle mit einer Membranoberfläche von 5 cm$^2$ (Fa. Amicon) gemischt. Die semipermeable Membran besaß eine Durchlässigkeit von 10.000 d. Der Überdruck im Gasvolumen oberhalb des Flüssigvolumens betrug 1,4 bis 1,6 atm. Jeder Strang der verwendeten BMV-mRNA enthielt die genetische Information für vier verschiedene Viruspeptide, deren Synthesezeit insgesamt und ohne Überlappung im Normalfall etwas weniger als fünf Minuten beträgt.

Die Zelle wurde von dem für die Translation erforderlichen Puffer durchströmt, wobei in sich zyklisch wiederholenden Zeitintervallen 5 µMol ATP zugeführt wurden. Nach jeweils fünf Stunden wurde das synthetisierte Produkt mit 5%iger Trichloressigsäure an einem Nitrozellulosefilter ausgefällt. Über eine Radioaktivitätsbestimmung wurde die Menge synthetisierter Polypeptidmoleküle pro Mol mRNA bestimmt.

Die Ergebnisse derartiger Bestimmungen mit Zyklusintervallen von jeweils 5, 10, 15, 20 bzw. 25 Minuten sind in Fig. 1 dem Resultat bei Verzicht auf eine variable Prozeßsteuerung gemäß dem in der EP A1 0312617 offenbarten Verfahren (Kontrolle) und denen der Versuchsreihe 2 gegenübergestellt.

Versuchsreihe 2: Variable Prozeßsteuerung durch zyklische Konzentrationsänderung von Protein- und Polynucleotidkomponenten

Es wurde der gleiche Versuchsansatz wie in Versuchsreihe 1 und dort beschrieben zugrundegelegt.

Anstelle nur einer Prozeßkomponente wie in Versuchsreihe 1 sollten die Konzentrationen sämtlicher, im zellfreien Translationsystem enthaltenen Prozeßkomponenten zyklisch geändert werden. Hierzu wurde das gesamte Flüssigvolumen der Ultrazentrifugationszelle innerhalb von 1 bis 3 Minuten auf 60 bis 70 % des Ausgangsvolumens konzentriert. Anschließend wurde in einer bestimmten Zeit von der dem Reaktionsvolumen entgegengesetzten Seite der Membran die für die Translation erforderliche Pufferlösung bis zum Erreichen des Ausgangsvolumens hinzugeführt. Der Prozeß der Konzentrierung und des Hinzuführens von Pufferlösung wurde innerhalb von fünf Stunden zyklisch wiederholt. Die synthetisierte Polypeptidmenge wurde analog der in der Versuchsreihe 1 angewendeten Methode bestimmt.

Ergebnisse dieser Bestimmungen über fünf verschiedene Steuerungszyklen hinweg sind ebenfalls in Fig. 1 dargestellt und den Resultaten bei Verzicht auf die variable Prozeßsteuerung (Kontrolle) und denen der Versuchsreihe 1 gegenübergestellt.

Die Resultate beider Versuchsreihen zeigen die deutliche Verbesserung der Peptidausbeuten gegenüber dem Verfahren ohne variable Prozeßsteuerung, bei dem die Prozeßkomponentenkonzentrationen kontinuierlich konstant gehalten werden und welches unter den bekannten Verfahren im zellfreien Translationssystem ohne Zweifel als das effektivste gelten kann. Obgleich die in den Versuchsreihen 1 und 2 vorgenommene Auswahl der Prozeßkomponenten wie auch die gewählten Steuerungszyklen lediglich nach verhältnismäßig groben Kriterien erfolgten und bei stärkerer Anlehnung an die in vivo-Translation noch weitaus differenzierter und feiner abgestimmt werden können, ergaben einige Verfahrensvarianten bereits um ca 40 % höhere Ausbeuten als das Kontrollverfahren.

Der Vergleich der Resultate innerhalb einer Versuchsreihe zeigt außerdem, daß der optimale Steuerungszyklus nicht notwendigerweise der einfachen Synthesezeit an einem Ribosom entsprechen muß. Wenn dem Steuerungszyklus beispielsweise die zweifachen (Versuchsreihe 1) bzw. die drei- bis vierfachen Synthesezeit (Versuchsreihe 2) zugrundegelegt wurde, konnten die Resultate gegenüber einer Zugrundelegung der einfachen Synthesezeit weiter verbessert werden.

Außerdem ist zu beachten, daß im realen polysomalen System die Bewegungsgeschwindigkeit der Ribosomen in verschiedenen Abschnitten der mRNA von der durchschnittlichen Geschwindigkeit für die gesamte mRNA abweichen kann, und aus diesem Grunde die Kurven für die Syntheseeffizienz in beiden Versuchsreihen keine ausgeprägten Maxima besitzen.

**Patentansprüche**

1. Verfahren zur Herstellung von Oligo- und Polypeptiden in einem zellfreien Translationssystem, welches neben einer Pufferlösung Prozeßkomponenten enthält, die an den einzelnen, jeweils eine Initiations-, Elongations- und Terminationsphase umfassenden ribosomalen Translationsprozessen im System unmittelbar oder mittelbar beteiligt sind, **dadurch gekennzeichnet,** daß die einzelnen ribosomalen Translationsprozesse phasengerichtet gesteuert und wenigstens teilweise synchronisiert werden durch in zeitlichen Abständen erfolgende Konzentrationsänderungen von wenigstens einer der Prozeßkomponenten, derart daß während wenigstens zwei der drei Phasen hinsichtlich wenigstens einer Prozeßkomponente für den Ablauf der jeweiligen Phase günstigere Bedingungen vorherrschen als in wenigstens einer der beiden anderen Phasen.

2.  Verfahren nach Anspruch 1, **dadurch gekennzeichnet,** daß die Konzentrationsänderungen im Rahmen eines sich regelmäßig wiederholenden Zyklus stattfinden.

3.  Verfahren nach einem der Ansprüche 1 oder 2, **dadurch gekennzeichnet,** daß als an den ribosomalen Translationsprozessen unmittelbar beteiligte Prozeßkomponenten Ribosomen und/oder ribosomale Untereinheiten und/oder Proteinkomponenten des Translationssystems und/oder Polynucleotide und/oder Substrate und/oder Endprodukte und/oder anorganische Ionen und/oder deren molekulare Komplexe und/oder Lösungsmittel verwendet werden.

4.  Verfahren nach Anspruch 3, **dadurch gekennzeichnet,** daß als Proteinkomponenten Initiations- und/oder Elongations- und/oder Terminationsfaktoren und/oder ribosomale Proteine verwendet werden.

5.  Verfahren nach Anspruch 3, **dadurch gekennzeichnet,** daß als Polynucleotide mRNA und/oder tRNA, insbesondere Aminoacyl- und Peptidyl-tRNA verwendet werden.

6.  Verfahren nach Anspruch 3, **dadurch gekennzeichnet,** daß als Substrate vorzugsweise ATP und/oder GTP und/oder Aminosäuren verwendet werden.

7.  Verfahren nach einem der Ansprüche 1 oder 2, **dadurch gekennzeichnet,** daß als an den ribosomalen Translationsprozessen mittelbar beteiligte Prozeßkomponenten insbesondere Komponenten des Transcriptionssystems und/oder des Nucleotidtriphosphat-regenerierenden Systems und/oder Enzyminhibitoren und/oder Enzymaktivatoren verwendet werden.

8.  Verfahren nach Anspruch 7, **dadurch gekennzeichnet,** daß als Prozeßkomponenten des Transcriptionssystems RNA-Polymerasen verwendet werden.

9.  Verfahren nach Anspruch 7, **dadurch gekennzeichnet,** daß als Komponenten des Nucleotidtriphosphat-regenerierenden Systems Phosphokreatin und/oder Phosphoenolpyruvat und/oder Phosphokreatinkinase und/oder Pyruvatkinase verwendet werden.

Fig. 1:       Anzahl [n der in fünf Stunden synthetisierten Polypep-
              tidkopierpro Mol mRNA des BMV bei unterschiedlicher
              Dauer (irMinuten) des Steuerungszyklus [t]

Kurve A:                 Anzahl der synthetisierten Polypeptidmole-
                         küle bei zyklischer Änderung der Konzentra-
                         tion des ATP (Versuchsreihe 1)

Kurve B:                 Anzahl der synthetisierten Polypeptidmole-
                         küle bei zyklischer Änderung der Konzentra-
                         tion sämtlicher im System enthaltenen Pro-
                         zeßkomponenten (Versuchsreihe 2)

Kontrolle:               Anzahl der synthetisierten Polypeptidmole-
                         küle bei konstanter Konzentration sämtli-
                         cher im System enthaltenen Prozeßkomponen-
                         ten (Kontrolle)